# EUROPEAN PATENT APPLICATION

(11) **EP 3 879 475 A1**
(43) Date of publication of application: **15.09.2021**
(21) Application number: 21167062.5
(22) Date of filing: 27.08.2014
(51) Int. Cl.: G06Q 10/10, G06Q 50/22, G06N 99/00, G06K 9/00, G06N 20/00, G16H 10/60, G16H 40/20, G16H 70/60

(54) **METHOD OF CLASSIFYING MEDICAL DOCUMENTS**

(30) Priority: 30.08.2013 US 201361871939 P
(62) Divisional of application: 14840414.8
(71) Applicant: 3M Innovative Properties Co., St. Paul, MN 55133-3427 (US)
(72) Inventor: ROBINSON, Samuel A., Saint Paul, MN Minnesota 55133-3427 (US); MARK, Jason M., Saint Paul, MN Minnesota 55133-3427 (US); GARRISON, Garri L., Saint Paul, mn Minnesota 55133-3427 (US); HARPER, Travis K., Saint Paul, MN Minnesota 55133-3427 (US)
(74) Representative: Vossius & Partner Patentanwälte Rechtsanwälte mbB

(57) **Abstract**

This disclosure describes systems, devices, and techniques for classifying medical documents. In one example, a method comprises receiving, with a computer system (110), one or more medical documents, wherein the one or more medical documents comprise one or more document regions (e.g., a document section, portion, or page), parsing, with the computer system (110), each of the one or more document regions, wherein the parsing comprises determining a number of times one or more features appear in each document region, and determining, by the computer system (110) and based on the parsing, a classification from a plurality of predetermined classifications for each of the one or more document regions.

## Description

### TECHNICAL FIELD

The invention relates to classifying medical documentation.

### BACKGROUND

In the medical field, accurate processing of records relating to patient visits to hospitals and clinics ensures that the records contain reliable and up-to-date information for future reference. Accurate processing may also be useful for medical systems and professionals to receive prompt and precise reimbursements from insurers and other payors. Some medical systems may include electronic health record (EHR) technology that assists in ensuring records of patient visits and files are accurate in identifying information needed for reimbursement purposes. These EHR systems generally have multiple specific interfaces into which medical professionals may input information about the patients and their visits.

### SUMMARY

In general, this disclosure describes systems and techniques for classifying medical documentation via one or more computing devices. The techniques and systems described herein can provide access to or enhance computer-assisted coding (CAC) by classifying medical documentation. In this manner, classifying medical documentation as described herein may improve and simplify the CAC process.

In one example, this disclosure describes a method of classifying medical document information, the method including receiving, with a computer system, one or more medical documents, wherein the one or more medical documents comprise one or more document regions, parsing, with the computer system, each of the one or more document regions, wherein the parsing comprises determining a number of times one or more features appear in each document region, and determining, by the computer system and based on the parsing, a classification from a plurality of predetermined classifications for each of the one or more document regions.

In another example, this disclosure describes a computerized system for classifying medical document information, the system comprising a processor and a memory, wherein the processor is configured to receive one or more medical documents, wherein the one or more medical documents comprise one or more document regions, parse each of the one or more document regions to determine a number of times one or more features appear in each document region, and determine, based on number of times one or more features appear in each document region, a classification from a plurality of predetermined classifications for each of the one or more document regions.

In another example, this disclosure describes a computerized system for classifying medical document information, the system comprising means for receiving one or more medical documents, wherein the one or more medical documents comprise one or more document regions, means for parsing each of the one or more document regions, wherein the means for parsing comprises means for determining a number of times one or more features appear in each document region, and means for determining, based on the parsing, a classification from a plurality of predetermined classifications for each of the one or more document regions.

The techniques of this disclosure may be implemented at least partially in hardware, such as a processor or discrete logic circuits. The techniques may also be implemented using aspects of software and/or firmware in combination with the hardware. If implemented at least partially in software or firmware, the software or firmware may be executed in one or more hardware processors, such as a microprocessor, application specific integrated circuit (ASIC), field programmable gate array (FPGA), or digital signal processor (DSP). The software that executes the techniques may be initially stored in a computer-readable storage medium and loaded and executed by the processor. The processor may execute modules to perform the techniques of this disclosure, and the modules may comprise combinations of software and hardware, e.g., software routines executing on the processor.

Accordingly, this disclosure also describes a computer-readable storage medium including instructions that, when executed, cause a processor to receive one or more medical documents, wherein the one or more medical documents comprise one or more document regions, parse each of the one or more document regions to determine a number of times one or more features appear in each document region, and determine, based on the number of times one or more features appear in each document region, a classification from a plurality of predetermined classifications for each of the one or more document regions.

In another example, this disclosure describes a method for analyzing medical document information, the method comprising receiving, with a computing system, one or more classifications associated with one or more respective document regions of a medical document, wherein each of the one or more classifications are selected from a plurality of predetermined classifications, generating, with the computing system and based on the classification of the respective document region, one or more medical codes for each of the classified document regions, and outputting, by the computing system, the generated one or more medical codes for each of the classified document regions of the medical document.

The details of one or more examples of the described systems, devices, and techniques are set forth in the accompanying drawings and the description below. Other features, objects, and advantages will be apparent from the description and drawings, and from the claims.

### BRIEF DESCRIPTION OF DRAWINGS

FIG. 1 is a block diagram illustrating an example of a stand-alone computer system configured for coding medical data consistent with this disclosure.
FIG. 2 is a block diagram illustrating an example of a stand-alone computer system configured for coding medical data consistent with this disclosure.
FIG. 3 is a block diagram illustrating an example of a distributed system configured for coding medical data consistent with this disclosure.
FIG. 4 is a flow diagram illustrating an example technique of this disclosure.
FIG. 5 is a flow diagram illustrating an example technique of this disclosure.
FIG. 6 is a flow diagram illustrating an example technique of this disclosure.

### DETAILED DESCRIPTION

This disclosure describes systems and techniques for classifying medical documentation via one or more computers. Typically, medical documentation may include an overview of a patient's health status and past care, along with any notes written by physicians, nurses, or other medical professionals. The documentation may take the form of a variety of different forms or records. In some medical systems that have included EHR technology, the EHR technology may require medical professionals to enter information into specific interfaces. These specific interfaces vary depending on the type of information to be entered and facilitate automated parsing by computer systems. These computing systems may assist in checking entered information for completeness and accuracy.

Some EHR systems may be integrated with computer systems that perform a process termed computer-assisted coding (CAC). Computer-assisted coding is a process for analyzing medical documents to ensure that correct medical codes have been identified based on information contained within the medical documentation. This information may have been inputted by medical professionals via the multiple specific interfaces provided to the medical professionals. In this manner, CAC may assist medical professionals, institutions, and other organizations in reviewing medical documentation. In some cases, the institutions or organization implementing EHR technologies will work with the technology provider of the EHR system to create the various interfaces for entering information. Additional infrastructure may then be needed to allow the computer systems performing CAC to communicate with the implemented EHR technology, as the interfaces may have different formats and protocols. This communication and interfacing between different technologies and technology platforms may present a challenge to building infrastructure that can extract and use the information in medical documentation for patients. Accordingly, implementing EHR technology can be expensive and time-consuming. These barriers may prevent various institutions or organizations from implementing EHR technology and, consequently, limit or entirely prohibit CAC processing in some circumstances.

Some organizations and institutions that have not implemented EHR technology may still generate and/or store medical files digitally, or at least have access to scanners and general purpose computers, which would allow the institutions to convert paper (e.g., handwritten or typed) records to digital records. The systems and techniques described herein describe, in one example, leveraging the "print" functionality of computers to output patients' electronic medical documentation, performing one or more processing steps on the documentation, and then performing CAC without the need for EHR technology and the infrastructure required for communication between the EHR technology platform and the CAC computer systems.

For example, this disclosure describes a method of classifying medical documents by receiving, with a computer system, one or more medical documents, wherein the one or more medical documents comprise one or more document regions. The method may further include parsing, with the computer system, each of the one or more document regions, wherein the parsing includes determining a number of times one or more features appear in each document region. The method may further comprise determining, by the computer system and based on the parsing, a classification from a plurality of predetermined classifications for each of the one or more document regions. In some examples, the method may further include generating, by the computer system and based at least in part on the one or more determined classifications, one or more medical codes. In this manner, the method may include processing received documents and performing CAC on received medical documentation without the need for EHR technology and the communication infrastructure required to implement the EHR technology.

As described herein, medical documents may include medical information related to a patient. Each medical document may be segmented, arranged, or otherwise generated into different sections, in some examples. Although, some medical documents may be a continuous document without any segmentation. In any case, each medical document may thus be comprised of one or more regions that may be identified and analyzed. A region may refer to a portion or subset of the information contained in the medical document. In one example, a region may refer to a section of the medical document separated by different headers or other markers. In another example, a region may refer to a page of the medical document, such as one of a plurality of digital pages or a representation of a piece of paper that was scanned into the system as part of a medical document and separated by digital page breaks. The examples described herein will refer to document pages as one example of regions of a medical document. However, the examples and techniques described may instead be applicable to any document region. Different document regions may be pre-defined as part of generating the initial medical document or dynamically-defined as part of the parsing process prior to classification.

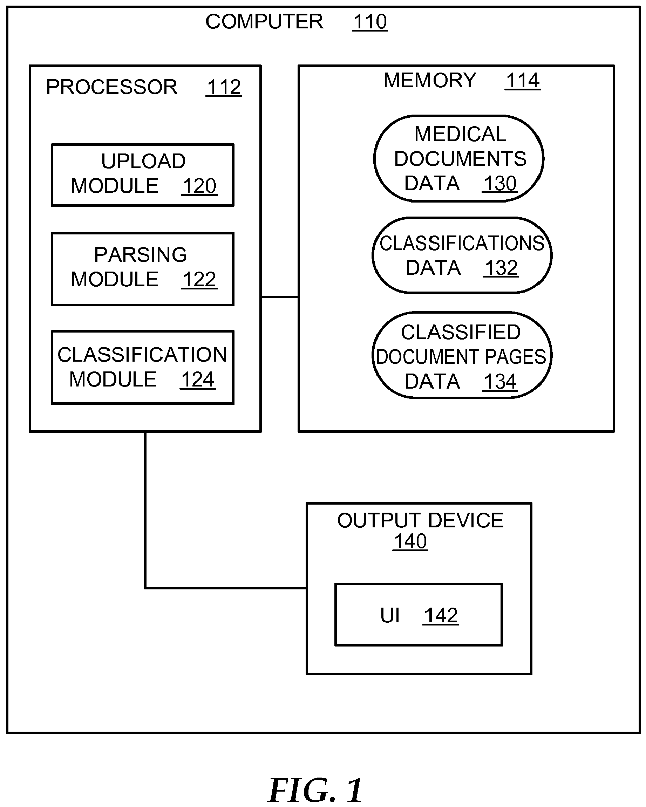

FIG. 1 is a block diagram illustrating an example of a stand-alone computerized system for coding medical data consistent with this disclosure. The system comprises computer 110 that includes processor 112, memory 114, and output device 140. Computer 110 may also include other components and modules related to the processes described herein and/or other processes. The illustrated components are shown as one example, but other examples may be consistent with various aspects described herein.

Output device 140 may be configured to output information to a user or other device. For example, output device 140 may include a display screen for presenting visual information to a user. In other examples, output device 140 may include one or more different types of devices for presenting information to a user. Memory 114 may be configured to store medical documents data 130, which may include data stored within documents such as patient medical records. Memory 114 may also be configured to store classifications data 132 and classified document pages data 134. Processor 112 may be configured to include upload module 120, parsing module 122, and classification module 124, each respective module configured to execute instructions and processes related to medical documents data 130 described herein. In some examples, classification module 124 may be configured to generate classified document pages data 134 that includes classified pages of medical documents. Although processor 112 is shown as including modules 120, 122, and 124, one or more of these modules may be separate from processor 112 in other examples. As described herein, a document page may be an example of a document region. Therefore, the techniques described herein may be used to classify document regions, not just segmentations of a medical document that may be referred to as different document pages.

Processor 112 may include a general-purpose microprocessor, a specially designed processor, an application specific integrated circuit (ASIC), a field programmable gate array (FPGA), a collection of discrete logic, and/or any type of processing device capable of executing the techniques described herein. In one example, memory 114 may be configured to store program instructions (e.g., software instructions) that are executed by processor 112 to carry out the techniques described herein. In other examples, the techniques described herein may be executed by specifically programmed circuitry of processor 112. Processor 112 may thus be configured to execute the techniques described herein. Processor 112, or any other processes herein, may include one or more processors.

Output device 140 may include a display screen and/or include other types of output capabilities. For example, output device 140 may include any number of visual (e.g., display devices, lights, etc.), audible (e.g., one or more speakers), and/or tactile feedback devices. In some examples, output device 140 may represent both a display screen (e.g., a liquid crystal display or light emitting diode display) and a printer (e.g., a printing device or module for outputting instructions to a printing device). In one example, upload module 120 may be configured to cause output device 140 to output user interface (UI) 142 for use by a user. UI 142 may be configured to allow users to view and select one or more medical documents from medical documents data 130, for example. UI 142 may further be configured to display classified document pages data 134. UI 142 may display one or more medical document pages and a generated classification for each page in some examples. Upload module 120 may be configured to transmit and receive data, such as medical documents data 130, classifications data 132, and classified document pages data 134 to and from memory 114. Upload module 120 may further transmit and receive data to and from output device 140, where output device 140 may display the data or a portion of data via UI 142. In some examples, UI 142 may be configured to receive user input and communicate user input to output device 140, processor 112 (including any of modules 120, 122, and 124), and/or to memory 114. Upload module 120 may additionally communicate data to and from parsing module 122 and classification module 124.

Medical documents data 130 may include any information relating to interaction between patients and medical facilities or professionals. In some examples, medical documents data 130 may also, or alternatively, include information collected or generated by medical device interaction with one or more patients. For example, during a visit to a facility or professional, a facility or medical professional may generate reports regarding a patient's health status, current treatments and outcomes, and/or results of any medical tests conducted for the patient. Generally, these medical documents may be grouped into a file identified as a health record for the specific patient. Each report may include multiple pages, portions, or sections, detailing the various aspects of the patient and/or the patient's visit. In some facilities, these patient health records may be kept as paper files. In some facilities, the paper records may be scanned and stored in a computer or computer memory as a digital health record that includes the medical documents. For example, upload module 120 may cause output device 140 to display, via UI 142, an interface that allows a user to scan and upload paper health records of a patient into computer 110 and store the digitized health record in memory 114 as part of medical documents data 130. Upload module 120 may additionally cause output device 140 to display, via UI 142, an interface configured to receive user input selecting one or more medical documents or files from medical documents data 130 to be classified by classification module 124.

Classifications data 132 may be stored by memory 114 and include various data (e.g., rules, features, instructions, algorithms) used to classify selected medical documents. For example, as will be described in more detail below, classifications data 132 may include features used in classifying selected medical documents. In some examples, these stored features may include words, phrases, characters, numbers, document titles, and other textual features that may be included in medical documents to be classified. Features may also include graphical components such as icons, symbols, or any other such identifiable items. In some examples, features may include various metadata related to medical documents. For example, features may include header information, text styles, page formatting, location of a document page relative to other document pages, position of various portions or sections within the medical document, and other metadata features. Classifications data 132 may additionally include various temporary data generated as a part of a document classification process, as will be explained in more detail below. In some examples, classifications data 132 may further include association information. The association information may be associations between various features and specific classifications that may be used as part of a classification process. For example, classifications data 132 may include an association between the phrase "history and physical" and the history and physical document classification. In some examples, the association may represent an addition or weighting factor that is used to correctly classify the portion of the medical document according to the identified features therein. Classifications data 132 may further include removable features. Removable features may include features, such as words characters, symbols, and/or phrases, that may be removed prior to or as part a classification process because the removable features may not assist the classification process. These removable features may be features of a medical document which do not affect the classification process or may interfere with correct classification of one or more portions of the document.

Classified document pages data 134 may include the output of a classification process that results in an association between each medical document page and a document classification. For example, classification module 124 may perform a classification process on one or more medical documents, where each medical document may comprise one or more pages (or sections or portions of the medical document). As a result of the process, classification module 124 may generate a document classification for each page of each medical document. Classification module 124 may then store the generated document classifications in memory 114 as classified document pages data 134 for each of the classified document pages or portions. In some examples, classification module 124 may generate the classified document pages data 134 as metadata attached to or otherwise associated with the respective pages of the medical document. In other examples, the classified document pages data 134 may be in the form of data linked to the respective medical document pages or otherwise available for later medical coding of the medical document.

As described above, UI device 142 may be configured to receive user input indicating to which medical documents to perform the classification process. Output device 140 may then transmit the indication via the processor 112 to the upload module 120, parsing module 122, and/or classification module 124. Upload module 120 may retrieve the indicated medical documents from memory 114 and transmit the medical documents to parsing module 122. In some examples, upload module 120 may employ a "print" function of computer 110 to generate one or more electronic images or text documents based on the medical documents. In other examples, upload module 120 may include a separate document generation engine configured to generate the document to which the classification process will be applied.

Parsing module 122 may then perform one or more pre-processing steps before classification module 124 performs the classification process. For example, parsing module 122 may perform one or more optical character recognition processes on the "printed" documents. In the case where one or more documents are in an image format, parsing module 122 may perform one or more optical character recognition processes on the image documents. The optical character recognition processes may convert the images of text from the medical document into text data, which may be a recognizable format to parsing module 122. Parsing module 122 may then scan the text data of each of the medical documents and remove any removable features. Some examples of removable features include words like "a", "and," the," and other articles not helpful to the classification process. In some examples, removable features may be any predetermined features that are stored in memory 114. Removal of the removable features may be performed prior to, during, or even after identifying any features for classification or generating counts of any of the identified features. In some examples, removing the removable features prior to and/or during the classification process may reduce analysis time and/or improve the accuracy of the classification process. Parsing module 122 may, in some examples, additionally generate counts of each identified feature in each page of each document. Counts of each respective feature may be stored as classifications data 132. In this manner, parsing module 122 may remove features that do not assist in classifying the medical documents (e.g. the removable feature) and generate counts for each feature indicating the number of times each feature appears in each document page. In some examples, parsing module 122 may store the counts of the features for each page as classifications data 132.

Classification module 124 may then process the documents and the data generated by parsing module 122 to generate a classification for each document page. In some examples, classification module 124 may generate a classification from a list of predetermined classifications. The list of predetermined classifications may include categories or types of medical information that may limit the number of applicable medical codes relevant to each classification. In this manner, the accuracy of later coding of the medical information may be improved with the aid of the context of the classification. In at least one example, the predetermined classifications may include a history and physical classification, an operative report classification, an emergency room classification, a progress notes classification, and a discharge summary classification. In other examples, the exact names and number of predetermined classifications may vary. For example, the types of classifications may be adjusted for types of facilities, medical practices, medical professionals, patients, or any other situation. In this manner, as few as two or three classifications may be used or a many as ten, twenty, or more classifications may be relevant to the medical documents to be classified.

In some examples, classification module 124 may build a statistical classifier based on the one or more features present in each document page, or even based on features that are not present. For example, classifications data 132 may include associations between each feature and a classification. As one example, classifications data 132 may store a feature "history and physical" that is associated with the history and physical classification. Classification module 124 may then sum all of the counts of features associated with each classification for each page to determine a total classification score for each classification for each page. Continuing the above example, if a document page had the feature "history and physical" appear three times on the page along with another feature associated with the history and physical classification that appeared four times, the total classification score for the history and physical classification may be summed as seven. Classification module 124 may perform a similar process for each classification for each document page. At the end, classification module 124 may generate a classification for each document page that corresponds to the highest classification score for that page.

The above example represents one example of how classification module 124 may generate a classification score. In other examples, classification module 124 may apply an addition factor or weighting factor (e.g., a multiplication factor), to the number of times a feature was identified, before determining each classification score. For example, the feature "history and physical" may be strongly associated with documents of the history and physical classification. Accordingly, classification module 124 may add a factor, or apply a weighting factor, to the count of how many times "history and physical" was determined to appear in a document page before adding together all the counts of features associated with the history and physical classification. These factors may help to correctly classify document pages based on stronger or weaker relevancy of each identified feature. Additionally, in some examples, features may be associated with multiple classifications and, accordingly, may increase the count of features associated with each of the classifications associated with the feature.

In some examples, classification module 124 may implement specific "rules." For example, classification data 132 may store one or more classification rules which classification module 124 apply. These classification rules may specify respective circumstances in which a document page should be classified as a specific one of the available classifications. For example, one classification rule may require classification module 124 to generate a history and physical classification for a document page in which the feature "history and physical" appears more than three times in the document page. In other examples, classification module 124 may use one or more statistical techniques to generate a classification for each document page. These statistical techniques may be used alone or in combination with other rules for classification. Example statistical techniques may include a Bayesian inference and the application of Fisher's combined probability test. One or more statistical techniques may be preselected or employed based on the type of medical document, type of identified features, number of potential classifications, or any other criteria. In some examples, classification module 124 may store the generated classifications for each page in memory 114 as classified document pages data 134.

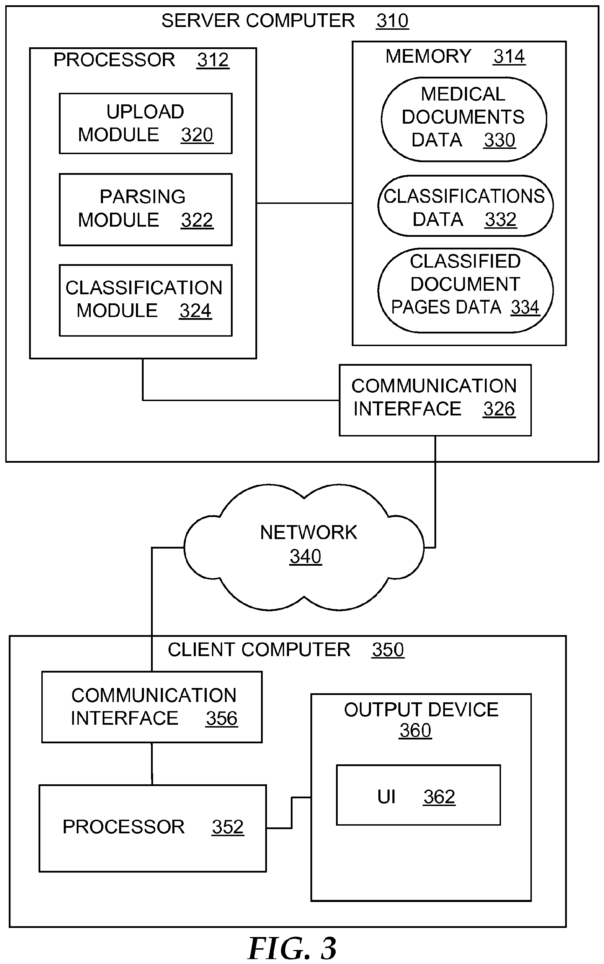

After computer 110 determines the classifications for each of the document pages of the medical document, computer 110 may be configured to transmit the medical document and/or the determined classifications to another device or system (e.g., a coding system or coding module) configured to code the document pages of the medical document. Computer 110 may include a communication module or other device that can transmit the classifications and/or medical document. The determined classifications and/or the medical document may be transmitted via a network (e.g., network 340 of FIG. 3) or other communication interface. In this manner, the classification process may be performed by a device or system different than a device or system configured to generate medical codes for the same medical document.

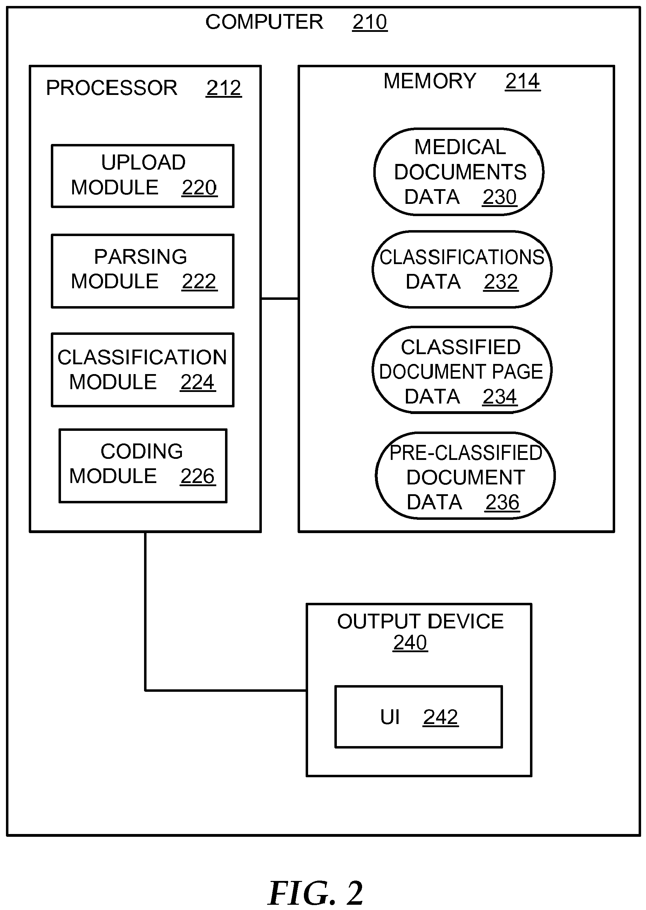

A computing system, different than computer 110, may be configured to receive the one or more classifications associated with one or more respective document pages of the medical document. As described herein each of the one or more classifications may be selected from a plurality of predetermined classifications. The computing system may include a coding module (e.g., similar to coding module 226 of FIG. 2) that is configured to generate, based on the classification of the respective document pages, one or more medical codes for each of the classified document pages.

In some examples, the coding module or other module may first parse the document pages or otherwise identify features or terms that may correspond to one or more predefined medical codes. The computing system that performs the coding may then output the generated one or more medical codes for each of the classified document pages of the medical document. This outputting step may include transmission of the medical codes to another system for further processing (e.g., a billing system or patient information management system).

In some examples, receiving the one or more classifications may include receiving the medical document. The medical document may thus include metadata, or some other type of information, that includes one or more classifications for each of the one or more document pages. The metadata may be a set of information identifying each document page and its respective classification that is stored as part of the medical document. The coding module may thus generate, based on the metadata, the medical codes for the document pages of the medical document. In other examples, the classifications may be stored as one or more separate files associated with the medical document via an identification number, file name, or some other linking information.

FIG. 2 is a block diagram illustrating an example of a stand-alone computerized system for coding medical data consistent with this disclosure. The system comprises computer 210 that includes processor 212, memory 214, and output device 240. Processor 212 may include upload module 220, parsing module 222, classification module 224, and coding module 226. Although processor 212 is shown as including modules 220, 222, 224, and 226, one or more of these modules may be separate from processor 112 in other examples. Memory 214 may include medical documents data 230, classifications data 232, classified documents page data 234, and pre-classified document data 236. Output device 240 may present UI 242 on a display device that is part of output device 240. Computer 210 may also include many other components. The illustrated components are shown merely to explain various aspects of this disclosure.

The modules and devices in FIG. 2 may be similar to similarly named devices and modules of FIG. 1 and may operate in similar fashion. For example, upload module 220 may be configured to perform a similar process to upload module 120. Parsing module 222 may be configured to perform a similar process to that described with respect to parsing module 122 of FIG. 1. Classification module 224 may perform a classification process similar to that described with respect to classification module 124 of FIG. 1.

One difference between the device of FIG. 1 and the device of FIG. 2 is the inclusion of coding module 226 in FIG. 2. In some examples, after classification module 224 performs a classification process, coding module 226 may perform the CAC process on the classified documents generated by classification module 224. For example, coding module 226 may be configured to parse the classified document pages and identify relevant medical codes. Examples of such healthcare codes include International Classification of Diseases (ICD) codes (versions 9 and 10), Current Procedural Technology (CPT) codes, Healthcare Common Procedural Coding System codes (HCPCS), and Physician Quality Reporting System (PQRS) codes. In some examples, coding module 226 may identify medical codes based on associations between specific features and specific medical codes.

In some examples, the classification of each document page also assists coding module 226 in identifying sources to determine the correct medical codes for the context of the document page. In other words, coding module 226 (or a coding module from a separate coding system) may utilize the classification of the document page to correctly determine the medical codes for that classified page. Using an example from the ICD-9 codeset, if a patient has had "myocardial infarction" in the past, the appropriate code that coding module 226 should generate to represent that myocardial infarction condition would be the 412 code (old myocardial infarction). However, if the medical document indicates that the condition is currently a present condition, the correct ICD-9 code to represent the current myocardial infarction would be the 410.71 code (subendocardial infarction, initial episode). This is one non-exclusive example of a case in which the correct classification of a document may assist coding module 226 in determining the appropriate code that should be applied to the conditions included in a medical document page. Other rules may use the classification data in other ways to assist coding module 226 in determining the correct medical codes for each document.

In some examples, coding module 226 may identify missing or incorrect medical codes based on the classification of the document page and the coding process. Coding module 226 may store a generated list of potentially missing or incorrect medical codes in memory 114. A medical professional, such as a medical coder, may then review this stored list and manually determine whether any of the listed medical codes are actually missing from or incorrect in the medical document. In this manner, coding module 226, or other module or system, may reduce the time needed for manual review by flagging or identifying potential errors in the coding process.

In the above described manner, computer 210 may be configured to assist medical facilities and professionals in correctly classifying medical documents. As described herein, the classification process performed prior to the coding process may further assist coding module 226 in performing CAC on the medical documents. Therefore, medical coding may be performed on medical documents for medical facilities without implementation of EHR technology that requires multiple interfaces and communication infrastructure between the interfaces and the EHR technology.

In some examples, upload module 220, in addition to performing a function similar to the function described with respect to upload module 120 in FIG. 1, may provide to a user, via output device 240 and UI 242, an interface that is configured to receive user input specifying additional data about a medical document or document page. For example, when selecting one or more documents or document pages to classify and/or process with CAC, a user may attach, via UI 242, metadata to each document or each page. For example, UI 242 may receive user input manually specifying a classification for each document or document page. Upload module 220 may store this classification in memory 214 as pre-classified document data 236. When performing a classification process, classification module 224 may retrieve information from pre-classified document data 236 and use the information to classify the indicated documents or document pages. In the example in which a user enters a specific classification, classification module 224 may then use the user classification to override other determinations and generate an association between the document or document page and the specified classification.

In at least one example, upload module 220 may output the classifications generated by classification module 224 to a user via output device 240 and UI 242. In some examples, upload module 220 may allow a user to input classifications for one or more of the document pages. Upload module 220 may then override the generated classifications of classification module 224 with the input classifications. In this manner, some examples of computer 210 may allow a user to check the results of classification module 224 and make any necessary adjustments.

In some examples, upload module 220 may output to a user, via output device 240 and UI 242, an option to "train" classification module 224. Processor 212, or a specific training module, may be configured to perform this training process. The training process may be helpful for when classification module 224 generates classifications for which a user often manually adjusts or alters. In other words, processor 212 may recognize manual changes to classifications of one or more pages and adjust the classification algorithm for future classification to incorporate the user's changes. If a user selects the training option, a user may select one or more documents along with a classification for each document page within the one or more documents. Upload module 220 may then store these documents along with the manually specified classifications in pre-classified document data 236. In some examples, classification module 224 may then parse each of the pre-classified documents and generate a listing of the identified features for each document page. Classification module 224 may then generate one or more rules, or adjust an addition factor or a weighting factor for one or more features, based on the identified features. For example, classification module 224 may determine that in the classification process described above, a certain weighting factor is too high for a particular feature because that factor would have caused classification module 224 to generate an incorrect classification for many pages that include that feature. This determination may be based on a comparison of the classification that classification module 224 would have generated and the user input specified classification. Although the above was described with a simple example, more generally, classification module 224 may be configured to adjust one or more of the statistical techniques employed to generate the classifications based on an analysis of features present in documents that a user has pre-classified. In this way, classification module 224 may develop a better indication of what a page that belongs to each classification "looks like," e.g., what features are included on the pages and what features are generally absent from the pages.

Additionally, in some examples, upload module 220 may allow a user to enter specific rules or manually adjust the addition or weighting factors. For example, if a document page that should be associated with a history and physical classification always has a feature "H&P" that is bold-faced, a user may enter a rule for classification module 224 to generate a history and physical classification for each document page that has a "H&P" feature that is bold-faced. Again, the above is just one specific example of a rule or adjustment a user may make to the classification module 224 and the classification process. More generally, upload module 220 may allow a user to modify any part of the described process. In this manner, processor 212 may train classification module 224 to more accurately classify medical document pages, which may further assist coding module 226 in correctly identifying medical codes.

FIG. 3 is a block diagram illustrating an example of a distributed system for coding medical data consistent with this disclosure. Although the processes described above with respect to FIGS. 1 and 2 were described as performed by a single device, the various described processes may be performed by multiple devices. Accordingly, FIG. 3 describes one example of how the processes may be split, or distributed, between multiple devices. However, other examples may include additional devices and/or split which processes are performed by which device in a different manner.

In at least one example, the system of FIG. 3 includes server computer 310 connected to client computer 350 via network 340. Server computer 310 may perform the techniques of this disclosure, but a user may interact with the system via client computer 350. In the example of FIG. 3, server computer 310 includes processor 312, memory 314, and communication interface 326. Processor 312 may comprise upload module 320, parsing module 322, and classification module 324. Although processor 312 is shown as including modules 320, 322, and 324, one or more of these modules may be separate from processor 112 in other examples. Memory 314 may include medical documents data 330, classifications data 332, and classified document pages data 334. Client computer 350 may include communication interface 356, processor 352, output device 360 and UI 362.

Network 340 may include a proprietary or non-proprietary network for packet-based communication. In one example, network 340 comprises the Internet, in which case communication interfaces 326 and 356 may include interfaces for communicating data according to transmission control protocol/internet protocol (TCP/IP), user datagram protocol (UDP), or the like. More generally, however, network 340 may include any type of communication network, and may support wired communication, wireless communication, fiber optic communication, satellite communication, or any type of techniques for transferring data between a source (e.g., server computer 310) and a destination (e.g., client computer 350).

Output device 360 may include a display screen, although this disclosure is not necessarily limited in this respect and other output devices may also be used. For example, output device 330 may generally represent both a display screen and a printer in some cases. Output device 360 may be similar to output device 160 of FIG. 1.

Processors 312 and 352 may each include a general-purpose microprocessor, a specially designed processor, an application specific integrated circuit (ASIC), a field programmable gate array (FPGA), a collection of discrete logic, or any type of processing device capable of executing the techniques described herein. In one example, memory 314 may store program instructions (e.g., software instructions) that are executed by processor 312 to carry out the techniques described herein. In other examples, the instructions may be executed by specifically programmed circuitry of processor 312. In these or other ways, processor 312 may be configured to execute the techniques described herein.

Similar to the stand-alone examples of FIG. 1 and 2, the distributed system example of FIG. 3 includes various modules configured to perform processes similar to process described above with respect to FIGS. 1 and 2. For example, output device 360 may comprise a display screen, and may also include other types of output capabilities. In some examples, upload module 320 may be configured to cause output device 360 to output UI 362. UI 362 may be configured to receive user input selecting one or more medical documents, for example from medical documents data 330. UI 362 may further present classified document pages data 334 to users for viewing, for example by displaying one or more medical document pages and a generated classification for each page. In this manner, upload module 320 may perform similar functions and processes as those described with respect to upload module 120 of FIG. 1. In some examples, upload module 320 may additionally perform similar functions and processes to upload module 220 described above with respect to FIG. 2. For example, upload module 320 may allow, in a manner similar to upload module 220 of FIG. 2, a user to specify additional data about a document or document page. For example, when selecting one or more documents or document pages to classify and/or process with CAC, a user may provide input requesting metadata be attached to each document or each page. In at least one example, upload module 320 may output the classifications generated by classification module 324 to a user through output device 360 and UI 342. Additionally, upload module 220 may also assist a user in performing "training" of classification module 324 in a similar manner to that described above with respect to upload module 220 of FIG. 2.

Additionally, parsing module 322 may perform similar processes and functions to parsing modules 122 and 222 as described above with respect to FIGS. 1 and 2. All of the data stored in memory 314, e.g., medical documents data 330, classifications data 332, and classified document pages data 334, may also all store similar data to that described with respect to medical documents data 130, classifications data 132, classified document pages data 134, medical documents data 230, classifications data 232, classified document pages data 234 of FIGS. 1 and 2. Classification module 324 may also perform similar processes and functions, for example classifying indicated medical documents and pages, as described previously with respect to classification modules 124 and 224 of FIGS. 1 and 2. One difference between FIGS. 1 and 2 and FIG. 3 is that the some modules for performing classification of medical documents reside on a different device than output device 360. However, in other examples, the specific location of each of modules 320, 322, and 324, and the location of data 330, 332, and 334 may be different than depicted in FIG. 3. For example, processor 352 of client computer 350 may include upload module 320. Upload module 320 may then communicate received data from UI 362 and output device 360 to server computer 310 through communication interface 356. Communication interface 356 may then communicate the data, according to one of various communication protocols, over network 340 to communication interface 326. Communication 326 may then communicate the received data to the appropriate module 320, 322, or 324, or to memory 314.

Although not depicted in FIG. 3, a distributed system may also include a module similar to coding module 226, as described previously with respect to FIG. 2. In at least one example, processor 312 may include such a coding module. Accordingly, in such examples, server computer 310 may operate to perform CAC on medical documents indicated by a user at client computer 350. A coding module may then communicate the results of the CAC process back to client computer 350 for display at output device 360 through UI 362. As with the other modules, in some examples, processor 352 may include a coding module, and client computer 350 may perform CAC. In other examples, another system or device may include a coding module and receive the classified medical documents.

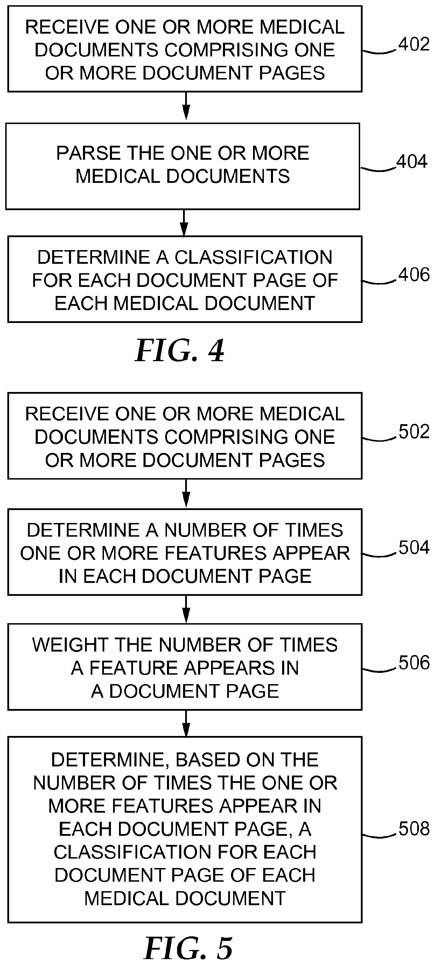

FIG. 4 is a flow diagram illustrating a technique consistent with this disclosure. FIG. 4 will be described from the perspective of computer 110 of FIG. 1, although the system of FIG. 2 or FIG. 3 or other systems could also be used to perform such techniques. As shown in FIG. 4, computer 110 receives one or more medical documents comprising one or more document pages (402). In some examples, a user may scan paper versions of the one or more document pages and instruct computer 110 to store the one or more scanned medical documents in memory 114. In some of these examples, upload module may generate and output at least a portion of UI 142, from which a user may instruct computer 110 to scan and store the medical documents. In this manner, computer 110 may receive user input instructing or commanding computing 110 to perform various processes. In other examples, a user may scan paper medical documents and digitally store, via a computing device, the scanned medical documents outside of any interaction with upload module 120. In other examples, a user may select and/or identify previously stored medical documents using output device 140 and UI 142. For example, upload module 120 may output UI 142. Within UI 142, a user may select from a list or folder of stored medical documents for processor 112 or any of modules 120, 122, and/or 124 to receive. Computer 110 may receive such user input selection and retrieve the selected medical documents.

Parsing module 122 may then parse the one or more medical documents (404). As described previously with respect to FIGS. 1 and 2, parsing module 122 may, in one example, leverage a "print" function of computer 110 in order to generate an image or text document representative of the respective one or more medical documents. In the case of an image document, parsing module 122 may further employ an optical character recognition process in order to convert the image in a format recognizable to parsing module 122. Parsing module 122 may, in some examples, remove any removable features from the one or more medical documents. Parsing module 122 may additionally generate a count of how many times each feature appears in each document page.

After generating a count of the features in each page, classification module 124 may then determine a classification for each document page of each medical document (406). Determining a classification may include employing one or more statistical techniques to manipulate the data generated by parsing module 122 to determine a classification for each page of each medical document. Some techniques used by classification module 124 to determine a classification have been described previously with respect to classification modules 124 and 224 of FIGS. 1 and 2, respectively.

FIG. 5 is a flow diagram illustrating a technique related to classifying medical documents. FIG. 5 will be described from the perspective of computer 110 of FIG. 1, although the system of FIG. 2 or FIG. 3 or other systems could also be used to perform such techniques. As shown in FIG. 5, computer 110 receives one or more medical documents comprising one or more document pages (502). In some examples, a user may scan paper versions of the one or more document pages and instruct computer 110 to store the one or more scanned medical documents in memory 114. In some of these examples, upload module may generate and output at least a portion of UI 142, from which a user may instruct computer 110 to scan and store the medical documents. In this manner, computer 110 may receive user input instructing or commanding computer 110 to perform various processes. In other examples, a user may scan paper medical documents and digitally store, via a computing device, the scanned medical documents outside of any interaction with upload module 120. In other examples, a user may select and/or identify previously stored medical documents using output device 140 and UI 142. For example, upload module 120 may output UI 142. Within UI 142, a user may select from a list or folder of stored medical documents for processor 112 or any of modules 120, 122, and/or 124 to receive. Computer 110 may receive such user input selection and retrieve the selected medical documents.

Using the selected medical document, parsing module 122 may then parse each document page of the document to identify features and determine a number of times one or more of the identified features appear in each document page (504). In addition, parsing module 122 may weight the number of times one or more of features were determined to appear in each document page (506). Weighting of one or more features may be performed to give more relevance to some features over other features when classifying a document. In other words, parsing module 122 may effectively rank the importance of some or all features for respective classifications using this weighting function.

In response to generating a count of the features in each page and weighting one or more of the features, classification module 124 may then determine a classification for each document page of each medical document (508). The determined classification for each page may be based on the number of times the one or more features appeared in the respective page and the weighting performed on the one or more features. In some examples determining the classifications may include employing one or more statistical techniques to manipulate the data generated by parsing module 122 to determine a classification for each page of each medical document. Some techniques used by classification module 124 to determine a classification have been described previously with respect to classification modules 124 and 224 of FIGS. 1 and 2, respectively.

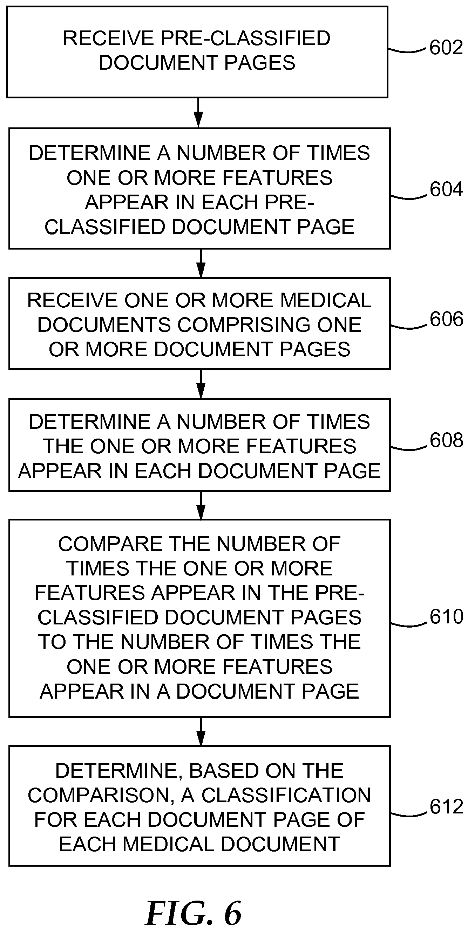

FIG. 6 is a flow diagram illustrating a technique related to using pre-classified documents to classify medical documents. FIG. 6 will be described from the perspective of computer 110 of FIG. 1, although the system of FIG. 2 or FIG. 3 or other systems could also be used to perform such techniques. FIG. 6 thus describes one example technique for classifying newly received medical document pages by comparing them to previously received pre-classified documents.

As shown in FIG. 6, computer 110 receives pre-classified document pages (602). In some examples, a user may scan in the one or more document pages and instruct computer 110 to store the one or more medical documents in memory 114. In some of these examples, upload module may output UI 142, from which a user may direct computer 110 to scan and store the medical documents. In other examples, a use may scan and store the medical documents outside of any interaction with upload module 120. In other examples, a user may indicate previously stored documents via output device 140 and UI 142. For example, upload module 120 may output UI 142. UI 142 may also receive user input selecting from a list or folder of stored medical documents for processor 112 or any of modules 120, 122, and/or 124 to receive. Additionally, a user may input, via UI 142 and output device 140, additional information about each document or document page, such as a classification. This additional information may be added by the user input. Alternatively, a pre-classified document page may have been already classified by computer 110, for example, and approved by user input. The approval may be in the form of receiving no user corrections prior to the classification being sent, an affirmative approval user input, or an approval after one or more correction inputs have been made to the classification.

Parsing module 122 may then determine a number of times one or more words appear in each pre-classified document page (604). For example, parsing module 122 may remove any removable features from each document page and identify the remaining features. Parsing module 122 may, for each document page, generate a count of each feature that appears in each document page.

Computer 110 may then receive one or more medical documents comprising one or more document pages (606). These medical documents are different than the pre-classified document pages. Computer 110 may receive the one or more medical documents in a similar manner to how computer 110 received the pre-classified document pages, or any other method of receiving documents as described herein. Parsing module 122 may then determine a number of times one or more features appear in each document page (608). Again, parsing module 122 may first remove any removable features before generating a count of how many times each feature appears in each document page.

Classification module 124 may then compare the number of times the one or more features appear in the pre-classified document pages to the number of times the one or more features appear in each received document page (610). In some examples, classification module 124 may employ a number of statistical techniques for the comparison, as described above. For example, classification module 124 may generate differences between the counts of features in pre-classified document pages with the counts of those same features in the respective received document pages. Then, classification module 124 may determine a total score for each classification based on the sum of the absolute values of the differences for the features between each of the pre-classified and received document pages where the score is indicative of the similarity between a received document page and pre-classified document pages of a particular classification. Finding a difference between the counts of features is one example of a statistical technique. More generally, classification module 124 may perform other statistical techniques as part of a comparison between the counts of the features present in the pre-classified document pages and the counts of the features in the received document pages.

Classification module 124 may then determine, based on the comparison, a classification for each document page of each medical document (612). In the example where classification module 124 determines a difference between the counts of the features present in the pre-classified document pages and the counts of the features in the received document pages, classification module 124 may determine a classification for each document page where the absolute values of the differences for the features between each of the pre-classified and received document pages is the lowest, which indicates a similarity in the number of features in a pre-classified document page and a received document page such that the classification of the pre-classified document page is applied to the similar received document page. In other examples, classification module 124 may generate the differences between counts and determine the classification as part of a single step instead of the separate steps 610 and 612 described above.

In some examples, classification module 124 may also compare the differences between the features of the pre-classified document pages to a threshold or apply a statistical technique. As long as the lowest absolute value difference between the documents is below the threshold or otherwise indicated as sufficiently similar, the classification of the similar pre-classified document page is applied to the received document page. If no pre-classified document pages are sufficiently similar to the received document page (e.g., the lowest absolute value difference is above the threshold), classification module 124 may initiate a training process using the non-matching received document page. For example, classification module 124 may request user input manually classifying the non-matching document page and/or correcting one or more of the pre-classified document pages. In response to receiving user input classifying the non-matching document page and/or correcting a pre-classified document page, classification module 124 may store the classified or corrected document pages as pre-classified document pages for later use in classifying additional documents. In other words, classification module 124 may update classification rules over time to improve the accuracy of the classification process.

As described herein, a document page may be an example of a document region. Therefore, the techniques described herein may be used to classify each region of a medical document. A document region may include one or more pages, one or more portions, or one or more sections of a medical document. Although medical documents may typically be segmented into "pages" that may or may not be limited to a specific type of medical information, the classification and coding techniques described herein are not limited to classification of segmented pages. Instead, different regions of a medical document may be separately classified, regardless of how the information of the medical document is visually segmented.

The techniques of this disclosure may be implemented in a wide variety of computer devices, such as servers, laptop computers, desktop computers, notebook computers, tablet computers, hand-held computers, smart phones, and the like. Any components, modules or units have been described to emphasize functional aspects and do not necessarily require realization by different hardware units. The techniques described herein may also be implemented in hardware, software, firmware, or any combination thereof. Any features described as modules, units or components may be implemented together in an integrated logic device or separately as discrete but interoperable logic devices. In some cases, various features may be implemented as an integrated circuit device, such as an integrated circuit chip or chipset.

If implemented in software, the techniques may be realized at least in part by a computer-readable storage medium comprising instructions that, when executed in a processor, performs one or more of the methods described above. The computer-readable storage medium may comprise a tangible computer-readable storage medium and may form part of a computer program product, which may include packaging materials. Example computer-readable storage media may include random access memory (RAM) such as synchronous dynamic random access memory (SDRAM), read-only memory (ROM), non-volatile random access memory (NVRAM), electrically erasable programmable read-only memory (EEPROM), FLASH memory, and magnetic or optical data storage media. The computer-readable storage medium may also comprise a non-volatile storage device, such as a hard-disk, magnetic tape, a compact disk (CD), digital versatile disk (DVD), Blu-ray disk, holographic data storage media, or other non-volatile storage device. The computer-readable storage medium may be referred to as a non-transitory medium. The term "non-transitory" may indicate that the storage medium is not embodied in a carrier wave or a propagated signal. In certain examples, a non-transitory storage medium may store data that can, over time, change (e.g., in RAM or cache).

The term "processor," as used herein may refer to any of the foregoing structure or any other structure suitable for implementation of the techniques described herein. In addition, in some aspects, the functionality described herein may be provided within dedicated software modules or hardware modules configured for performing the techniques of this disclosure. Even if implemented in software, the techniques may use hardware such as a processor to execute the software, and a memory to store the software. In any such cases, the computers described herein may define a specific machine that is capable of executing the specific functions described herein. Also, the techniques could be fully implemented in one or more circuits or logic elements, which could also be considered a processor.

Various examples have been described. These and other examples are within the scope of the following claims.

The following aspects are preferred embodiments of the present invention:
1. A method of classifying medical document information, the method comprising:
   receiving, with a computer system, one or more medical documents, wherein the one or more medical documents comprise one or more document regions;
   parsing, with the computer system, each of the one or more document regions, wherein the parsing comprises determining a number of times one or more features appear in each document region; and
   determining, by the computer system and based on the parsing, a classification from a plurality of predetermined classifications for each of the one or more document regions.
2. The method of aspect 1, further comprising transmitting, by the computer system, the classifications for each of the one or more document regions to a coding system configured to generate or more medical codes based at least in part on the one or more determined classifications.
3. The method of aspect 1, wherein parsing each of the one or more document regions further comprises weighting the number of times each of the one or more features appear in each document region.
4. The method of aspect 1, wherein determining the classification for each of the one or more document regions comprises:
   generating, for each of the one or more document regions and based on the number of times the one or more features appear in the respective document region, a classification score associated with each of the predetermined classifications; and
   selecting, by the computer system and based on the associated classification scores, one of the predetermined classifications for each of the one or more document regions.
5. The method of aspect 4, wherein the classification score is a probability that medical information of the document region belongs to the predetermined classification, and
   wherein selecting one of the predetermined classifications comprises selecting the classification associated with the highest probability that the document region belongs to the predetermined classification.
6. The method of aspect 1, wherein parsing each of the one or more document regions further comprises removing one or more removable features from the respective document region prior to or during determining the number of times one or more features appear in the respective document region.
7. The method of aspect 1, wherein the predetermined classifications comprise:
   a history and physical classification;
   an operative reports classification;
   an emergency room classification;
   a progress notes classification; and
   a discharge summary classification.
8. The method of aspect 1, wherein parsing each of the one or more document regions further comprises:
   processing the one or more document regions according to one or more techniques, the one or more techniques comprising:
   natural language processing techniques;
   optical character recognition techniques; and
   statistical analysis techniques.
9. The method of aspect 1, further comprising:
   receiving, by the computer system, one or more pre-classified document regions; and
   parsing, by the computer system, each of the one or more pre-classified document regions to determine a number of times the one or more features appear in each pre-classified document region,
   wherein determining the classification from a plurality of predetermined classifications for each of the one or more document regions comprises comparing, by the computer system, the number of times the one or more features appear in each of the pre-classified document regions to the number of times the one or more features appear in each of the respective document regions.
10. A computerized system for classifying medical document information, the system comprising a processor and a memory, wherein the processor is configured to:
   receive one or more medical documents, wherein the one or more medical documents comprise one or more document regions;
   parse each of the one or more document regions to determine a number of times one or more features appear in each document region; and
   determine, based on number of times one or more features appear in each document region, a classification from a plurality of predetermined classifications for each of the one or more document regions.
11. The system of aspect 10, wherein the processor is further configured to transmit the classifications for each of the one or more document regions to a coding system configured to generate one or more medical codes based at least in part on the one or more determined classifications.
12. The system of aspect 10, wherein the processor is further configured to weight the number of times each of the one or more features appear in each of the one or more document regions.
13. The system of aspect 10, wherein to determine a classification for each of the one or more document regions, the processor is further configured to:
   generate, for each of the one or more document regions and based on the number of times the one or more features appear in the respective document region, a classification score associated with each of the predetermined classifications; and
   select, based on the associated classification scores, one of the predetermined classifications for each of the one or more document regions.
14. The system of aspect 13, wherein the classification score is a probability that medical information of the document region belongs to the predetermined classification, and
   wherein to select one of the predetermined classifications, the processor is configured to select the classification associated with the highest probability that the document region belongs to the predetermined classification.
15. The system of aspect 10, wherein the predetermined classifications comprise:
   a history and physical classification;
   an operative reports classification;
   an emergency room classification;
   a progress notes classification; and
   a discharge summary classification.
16. The system of aspect 10, wherein the processor is further configured to process each of the one or more document regions according to one or more techniques, the one or more techniques comprising:
   natural language processing techniques;
   optical character recognition techniques; and
   statistical analysis techniques.
17. The system of aspect 10, wherein the processor is further configured to:
   receive one or more pre-classified document regions;
   parse each of the one or more pre-classified document regions to determine a number of times one or more features appear in each pre-classified document region; and
   compare the number of times one or more features appear in each pre-classified document region to the number of times any same one or more features appear in each of the respective one or more document regions to determine the classification of each of the one or more document regions.
18. A computer-readable storage medium comprising instructions that, when executed, cause a processor to:
   receive one or more medical documents, wherein the one or more medical documents comprise one or more document regions;
   parse each of the one or more document regions to determine a number of times one or more features appear in each document region; and
   determine, based on the number of times one or more features appear in each document region, a classification from a plurality of predetermined classifications for each of the one or more document regions.
19. A method for analyzing medical document information, the method comprising:
   receiving, with a computing system, one or more classifications associated with one or more respective document regions of a medical document, wherein each of the one or more classifications are selected from a plurality of predetermined classifications;
   generating, with the computing system and based on the classification of the respective document region, one or more medical codes for each of the classified document regions; and
   outputting, by the computing system, the generated one or more medical codes for each of the classified document regions of the medical document.
20. The method of aspect 19, wherein receiving the one or more classifications comprises receiving the medical document, the medical document comprising metadata that includes one or more classifications for each of the one or more document regions, and wherein generating the one or more medical codes comprises generating, based on the metadata, the one or more medical codes for each of the classified document regions.

## Claims

1. A computerized system for classifying medical document information, the system comprising a processor and a memory, wherein the processor is configured to:
receive, from the memory, one or more medical documents in an electronic image format, wherein the one or more medical documents comprise one or more document regions;
parse the one or more document regions to determine a number of times one or more features appear in each document region, wherein the one or more feature comprise features selected from a group consisting of graphical symbols and metadata related to the one or more medical documents selected from header information, text styles, page formatting, location of a document page relative to other document pages, position of portions or sections within the medical document; and
determine, based on the number of times one or more features appear in each document region, a classification from a plurality of predetermined classifications for each of the one or more document regions, wherein to determine the classification for each of the one or more document regions, the processor is further configured to:
generate, for each of the one or more document regions and based on the number of times the one or more features appear in the respective document region, a classification score associated with each of the predetermined classifications;
if the one or more document regions are sufficiently similar to one of the predetermined classifications when applying a threshold comparison, select, based on the associated classification scores, one of the predetermined classifications for each of the one or more document regions;
if the one or more document regions are not sufficiently similar to any of the predetermined classifications when applying the threshold comparison, initiate a training process to update classification rules.

2. The system of claim 1, wherein the processor is configured such that the initiated training process comprises:
requesting user input classifying a non-matching document region that is not sufficiently similar to any of the predetermined classifications and/or correcting a pre-classified document region;
updating, in response to receipt of the user input classifying the non-matching document region and/or correcting the pre-classified document region, classification rules to improve an accuracy of the classification process.

3. The system of claim 1, wherein the processor is configured such that the initiated training process comprises:
requesting user input classifying a non-matching document region that is not sufficiently similar to any of the predetermined classifications and/or correcting a pre-classified document region;
storing, in response to receipt of the user input classifying the non-matching document region and/or correcting the pre-classified document region, the corrected pre-classified document region for later use in classifying additional documents.

4. The system of any one of claims 1 to 3, wherein the system is a distributed computing system comprising:
a server computer (310) comprising the memory and the processor,
a client computer (350),
a network (340) via which the server computer (310) is connected to the client computer.

5. The system of any one of the preceding claims, wherein the processor comprises an application specific integrated circuit or a field programmable gate array.

6. The system of any one of the preceding claims, wherein the processor is further configured to transmit the classifications for each of the one or more document regions to a coding system configured to generate one or more medical codes based at least in part on the one or more determined classifications.

7. The system of any one of the preceding claims, wherein the processor is further configured to weight the number of times each of the one or more features appear in each of the one or more document regions.

8. The system of any one of the preceding claims, wherein the classification score is a probability that medical information of the document region belongs to the predetermined classification, and
wherein to select one of the predetermined classifications, the processor is configured to select the classification associated with the highest probability that the document region belongs to the predetermined classification.

9. The system of any one of the preceding claims, wherein the predetermined classifications comprise:
a history and physical classification;
an operative reports classification;
an emergency room classification;
a progress notes classification; and
a discharge summary classification.

10. The system of any one of the preceding claims, wherein the processor is further configured to process each of the one or more document regions according to one or more techniques, the one or more techniques comprising natural language processing techniques.

11. The system of any one of the preceding claims, wherein the processor is further configured to:
receive one or more pre-classified document regions;
parse each of the one or more pre-classified document regions to determine a number of times one or more features appear in each pre-classified document region; and
compare the number of times one or more features appear in each pre-classified document region to the number of times any same one or more features appear in each of the respective one or more document regions to determine the classification of each of the one or more document regions.

12. The system of any one of the preceding claims, wherein the processor is further configured to process each of the one or more document regions according to one or more techniques, the one or more techniques comprising statistical analysis techniques.

13. The system of any one of the preceding claims, wherein the processor is further configured to:
train a classification algorithm by recognizing manual changes to one or more classifications of a first document region of the one or more medical documents; and
determine, based on the trained classification algorithm and the number of times the one or more features appear in at least one document region of the one or more document regions, the classification for a second document region of the one or more medical documents.
